# EUROPEAN PATENT APPLICATION

(11) **EP 4 837 909 A1**
(43) Date of publication of application: **11.11.2026**
(21) Application number: 25736343.2
(22) Date of filing: 03.01.2025
(51) Int. Cl.: C07D 405/14, C07D 405/04

(54) **METHOD FOR PREPARING ALK INHIBITOR COMPOUND AND INTERMEDIATE THEREOF**

(30) Priority: 05.01.2024 CN 202410019568
(71) Applicant: Ascentage Pharma (Suzhou) Co., Ltd., Suzhou, Jiangsu 215127 (CN); Ascentage Pharma Group Corp Limited, Hong Kong 999077 (HK)
(72) Inventor: WU, Tianzhu, Suzhou, Jiangsu 215127 (CN); FENG, Jianqing, Suzhou, Jiangsu 215127 (CN); XU, Feng, Suzhou, Jiangsu 215127 (CN); MIAO, Yunfeng, Suzhou, Jiangsu 215127 (CN)
(74) Representative: AWA Denmark A/S
(86) International application number: PCT/CN2025/070457
(87) International publication number: WO 2025/146139

(57) **Abstract**

Disclosed in the present invention is a method for preparing an ALK inhibitor compound and an intermediate thereof. Specifically, the present invention provides a method for preparing compound A9, comprising the following reaction steps: (1) carrying out a coupling reaction on "compound A2 or a salt thereof" and "compound B3 or a salt thereof" in a solvent in the presence of a metal catalyst, a ligand, and a base to obtain compound A4, wherein the metal catalyst is a palladium catalyst; and (2) carrying out a coupling reaction on compound A4 and compound A8 in a solvent in the presence of an acid to obtain compound A9. The preparation method of the present invention has the advantages of proper reaction steps, simple operation, cheap and easily available reaction raw materials, environmental friendliness, simple post-treatment, low safety risk, reduced production cycle and production cost, etc.

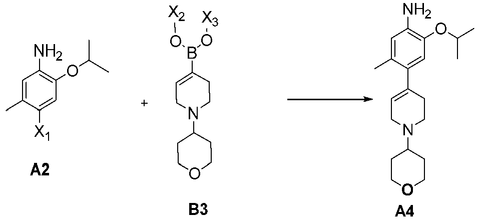

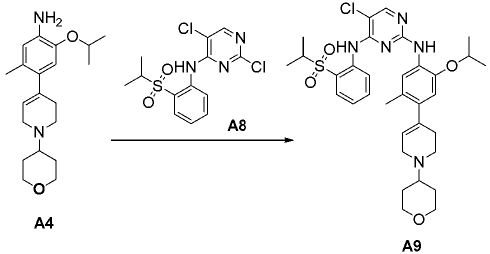

## Description

The present application claims priority to Chinese Patent Application No. 2024100195681 filed on January 5, 2024, which is incorporated herein by reference in its entirety.

### TECHNICAL FIELD

The present disclosure relates to a preparation method for an ALK inhibitor compound and an intermediate thereof.

### BACKGROUND

Anaplastic lymphoma kinase (ALK) is a member of the insulin receptor superfamily of receptor tyrosine kinases and has been implicated in the development of hematopoietic and non-hematopoietic tumors. Aberrant expression of the full-length ALK receptor protein in neuroblastoma and glioblastoma has been reported, and ALK fusion proteins occur in anaplastic large cell lymphoma. Studies of ALK fusion proteins have also suggested the possibility of new therapeutic treatments for patients with ALK-positive malignancies. Small-molecule ALK inhibitors have therapeutic potential for the treatment of diseases and conditions in which ALK plays a role, and the diseases and conditions include cancer.

In the prior art, a preparation method for compound A9 as an ALK inhibitor has been disclosed, and the synthetic route is shown as follows:

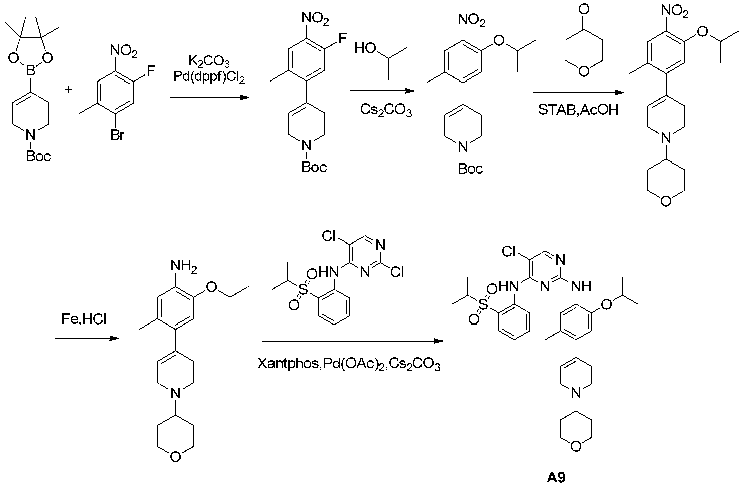

The route has a relatively long production cycle, requires a relatively large amount of palladium catalyst, results in relatively high production costs, and involves a complex synthetic route.

### SUMMARY

The technical problem to be solved by the present disclosure is that the existing preparation methods for ALK inhibitors have a relatively long production cycle, relatively high production costs, and complex synthetic routes. The present disclosure provides a preparation method for an ALK inhibitor compound and an intermediate thereof. The preparation method has the advantages of appropriate reaction steps, simple and convenient operation, inexpensive and readily available reaction starting materials, environmental friendliness, simple and convenient work-up, low safety risk, and reduced production cycle and production cost.

The present disclosure provides a preparation method for compound A9, and the preparation method comprises the following reaction steps: (1) performing a coupling reaction between "compound A2 or a salt thereof" and "compound B3 or a salt thereof" in a solvent in the presence of a metal catalyst, a ligand, and a base to obtain compound A4, wherein the metal catalyst is a palladium catalyst; 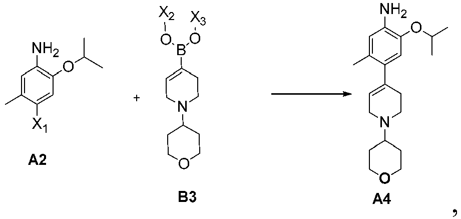 (2) performing a coupling reaction between compound A4 and compound A8 in a solvent in the presence of an acid to obtain compound A9; 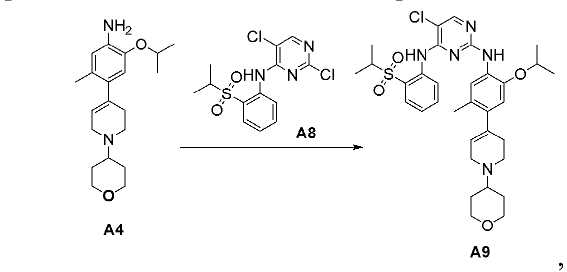
X₁ is halogen, e.g., F, Cl, Br, or I;
X₂ and X₃ are each independently H or C₁₋₆ alkyl; or, X₂ and X₃ are linked to form -C(CH₃)₂-C(CH₃)₂-.

In some embodiments, X₁ is Cl.

In some embodiments, the palladium catalyst is palladium(II) acetate, tetrakis(triphenylphosphine)palladium, or bis(triphenylphosphine)palladium (II) chloride, preferably palladium(II) acetate.

In some embodiments, X₂ and X₃ are each independently H or C₁₋₆ alkyl.

In some embodiments, X₂ and X₃ are linked to form -C(CH₃)₂-C(CH₃)₂-, that is, 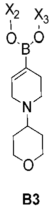 is

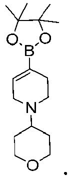

In some embodiments, in step (1), the solvent is one or more of an alcohol solvent, an ether solvent, an aromatic solvent, a nitrile solvent, a sulfoxide solvent, an amide solvent, and water, e.g., a combination of an alcohol solvent and water, a combination of an ether solvent and water, a combination of an aromatic solvent and water, a combination of a nitrile solvent and water, a combination of an amide solvent and water, an aromatic solvent, a nitrile solvent, a sulfoxide solvent, or an amide solvent, preferably a combination of an alcohol solvent and water, a combination of an aromatic solvent and water, a combination of a nitrile solvent and water, or a combination of an amide solvent and water.

In some embodiments, in step (1), the alcohol solvent is isopropanol.

In some embodiments, in step (1), the ether solvent is dioxane and/or methyl ether.

In some embodiments, in step (1), the aromatic solvent is toluene.

In some embodiments, in step (1), the nitrile solvent is acetonitrile.

In some embodiments, in step (1), the sulfoxide solvent is dimethyl sulfoxide.

In some embodiments, in step (1), the amide solvent is N,N-dimethylformamide. In some embodiments, in step (1), when the solvent is a combination of an alcohol solvent and water, the mass ratio of the alcohol solvent to the water is (4-20):1, preferably 11.9:1.

In some embodiments, in step (1), the salt of compound A2 is a hydrochloride form of compound A2.

In some embodiments, in step (1), when the salt of compound A2 is the hydrochloride form of compound A2, the molar ratio of compound A2 to hydrochloric acid is 1:1.

In some embodiments, in step (1), the "salt of compound A2" and "compound B3" are subjected to a coupling reaction to obtain compound A4.

In some embodiments, in step (1), compound B3 is in a free form.

In some embodiments, in step (1), the base is an alkali metal carbonate and/or an alkali metal phosphate, for example, potassium carbonate and/or potassium phosphate, preferably potassium phosphate.

In some embodiments, in step (1), the ligand is a bidentate phosphine ligand or a biaryl phosphine ligand, e.g., BrettPhos ( 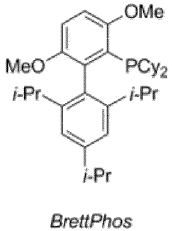 ), RuPhos ( 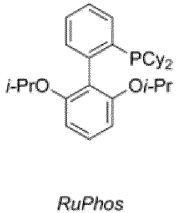 ), Xphos ( 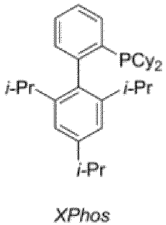 ), SPhos ( 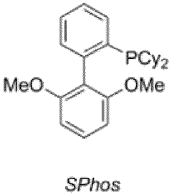 ), Bippyphos ( 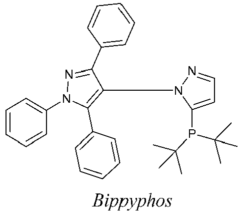 ), CyPF-t-Bu ( 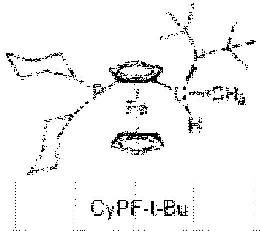 ), Josiphos ( 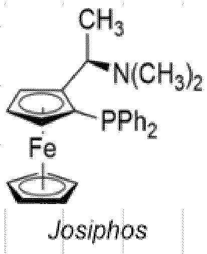 ), BINAP ( 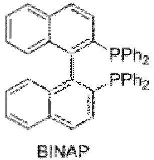 ), tBuXphos ( 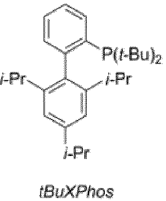 ), Me₄tBuXphos ( 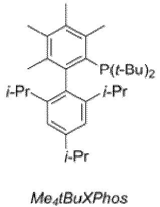 ), tBuBrettPhos ( 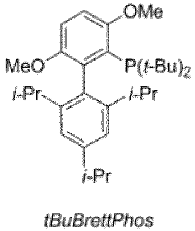 ), DavePhos ( 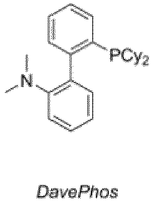 ), JohnPhos ( 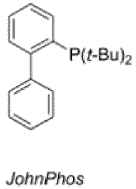 ), JackiePhos ( 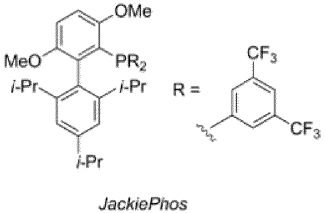 ), (R)-(S)-Cy2PF-PtBu2 ( 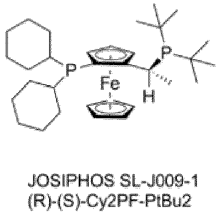 ), XantPhos ( 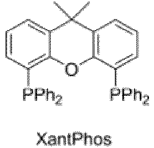 ), or PCy₃HBF₄, preferably RuPhos, Sphos, Xphos, or PCy₃HBF₄, more preferably RuPhos.

In some embodiments, in step (1), the molar ratio of compound B3 to compound A2 is (1-1.1):1, e.g., 1.1:1, 1.05:1, or 1.0:1, preferably 1.1:1.

In some embodiments, in step (1), the molar ratio of the metal catalyst to compound A2 is (0.01-0.02):1, e.g., 0.01:1 or 0.02:1, preferably 0.01:1.

In some embodiments, in step (1), the molar ratio of the base to compound A2 is (3.0-5.0):1, e.g., 3.0:1, 4.0:1, or 5.0:1, preferably 3.0:1.

In some embodiments, in step (1), the molar ratio of the ligand to compound A2 is 0.01:1-0.03:1, preferably 0.02:1.

In some embodiments, in step (1), when the solvent is a combination of an alcohol solvent and water, the volume-to-molar ratio of the water to compound A2 is 0.20 L/mol-0.40 L/mol, preferably 0.35 L/mol.

In some embodiments, in step (1), the coupling reaction is performed at a reaction temperature of 80-120 °C, preferably 75-85 °C.

In some embodiments, in step (1), the coupling reaction is performed for 4-8 hours, preferably 6 hours.

In some embodiments, in step (1), the coupling reaction is performed under an inert gas atmosphere, for example, under nitrogen atmosphere.

In some embodiments, in step (1), the coupling reaction further comprises a palladium removal step, preferably using N-acetyl-L-cysteine and activated carbon simultaneously for palladium removal; the molar ratio of N-acetyl-L-cysteine to compound A2 is preferably 0.1:1; the mass percentage of the activated carbon in the reaction system is preferably 10%.

In some embodiments, in step (1), the coupling reaction comprises the following work-up steps: liquid separation (e.g., liquid separation using purified water), concentration (e.g., first concentrating the organic phase and then adding ethyl acetate and concentrating), extraction (e.g., using ethyl acetate and water), palladium removal, filtration, washing (e.g., washing using an aqueous sodium carbonate solution and saturated brine), concentration (e.g., first concentrating, then adding methyl tert-butyl ether and n-heptane and concentrating, and then adding n-heptane and concentrating), and recrystallization (e.g., recrystallization using n-heptane and/or methyl tert-butyl ether).

In some embodiments, in step (2), the solvent is an alcohol solvent, for example, isopropanol.

In some embodiments, in step (2), the acid is an organic strong acid or an inorganic strong acid, wherein the organic strong acid may be 2,4,6-trinitrophenol (picric acid), 2,4,6-trinitrobenzoic acid (pyro-picric acid), trifluoroacetic acid, trichloroacetic acid, methanesulfonic acid, benzenesulfonic acid, cyclohexylthiolsulfonic acid, or p-toluenesulfonic acid; the inorganic strong acid may be concentrated hydrochloric acid, an organic solution of hydrogen chloride (e.g., a solution of hydrogen chloride in isopropanol, hydrogen chloride in ethyl acetate, hydrogen chloride in methanol, hydrogen chloride in ethanol, or hydrogen chloride in dioxane), perchloric acid, hydroiodic acid, hydrobromic acid, sulfuric acid, or nitric acid, preferably concentrated hydrochloric acid.

In some embodiments, in step (2), the molar ratio of compound A8 to compound A4 is (1-1.5):1, e.g., 1.05:1.

In some embodiments, in step (2), the molar ratio of the acid to compound A4 is (1-1.5):1, e.g., 1.2:1.

In some embodiments, in step (2), the molar-to-volume ratio of compound A4 to the solvent is 1:(6-15) mol/L, e.g., 1:8 mol/L.

In some embodiments, in step (2), the coupling reaction is performed at a temperature of 70-90 °C, preferably 75-85 °C.

In some embodiments, in step (2), the coupling reaction is performed under an inert gas atmosphere, for example, under nitrogen atmosphere.

In some embodiments, in step (2), the coupling reaction is performed for 12-24 hours, e.g., 24 hours.

In some embodiments, in step (2), the coupling reaction further comprises the following work-up steps: crystallization (e.g., crystallization using ethyl acetate), filtration, washing (e.g., washing using ethyl acetate, dichloromethane, and 10% sodium carbonate solution), liquid separation, washing an organic phase (e.g., washing using 10% sodium carbonate solution), concentration under reduced pressure, crystallization (e.g., crystallization using acetonitrile), and drying.

In some embodiments, the preparation method for compound A9 further comprises the following step: performing a reduction reaction of compound A1 in a solvent in the presence of a reducing agent to obtain compound A2 or the salt thereof; 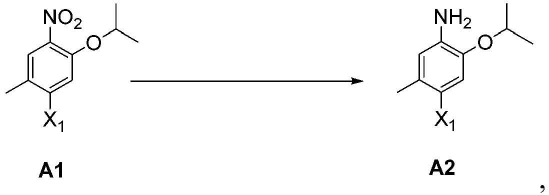 wherein X1 is F, Cl, Br, or I.

In some embodiments, compound A1 is subjected to a reduction reaction to obtain the salt of compound A2.

In some embodiments, the reducing agent is iron powder and concentrated hydrochloric acid, stannous chloride dihydrate and concentrated hydrochloric acid, hydrazine hydrate and ferric chloride, Pd/C and H₂, or Pt/C and H₂, preferably Pt/C and H₂.

In some embodiments, in the reduction reaction, the hydrogen pressure of the reaction system is 0.05-0.6 MPa, preferably 0.2-0.4 MPa, more preferably normal pressure.

In some embodiments, in the reduction reaction, the reaction system is first purged with nitrogen and then purged with hydrogen.

In some embodiments, in the reduction reaction, the solvent is an organic solvent or a combination of an organic solvent and water, and the organic solvent is an ether solvent (e.g., tetrahydrofuran), an ester solvent (e.g., ethyl acetate), or an alcohol solvent (e.g., methanol or isopropanol); preferably, the solvent is an ester solvent or a combination of an ester solvent and water; more preferably, the solvent is ethyl acetate or a combination of ethyl acetate and water.

In some embodiments, in the reduction reaction, the molar-to-volume ratio of compound A1 to the organic solvent is (1-3):10 mol/L, e.g., 2:10 mol/L.

In some embodiments, in the reduction reaction, when the solvent is a combination of an organic solvent and water, the mass ratio of the water to compound A1 is 2%-4%, e.g., 3%.

In some embodiments, in the reduction reaction, the Pt/C is 3% Pt/C.

In some embodiments, in the reduction reaction, the mass percentage of the reducing agent to compound A1 is 4%-6%, e.g., 5%.

In some embodiments, in the reduction reaction, the mass percentage of the Pt/C to compound A1 is 4%-6%, e.g., 5%.

In some embodiments, the salt of compound A2 is the hydrochloride form of compound A2.

In some embodiments, when the salt of compound A2 is the hydrochloride form of compound A2, the molar ratio of compound A2 to hydrochloric acid is 1:1.

In some embodiments, the reduction reaction is performed at a temperature of 40-70 °C, e.g., 40-50 °C.

In some embodiments, the reduction reaction is performed for 18-20 hours, e.g., 20 hours.

In some embodiments, the reduction reaction further comprises the following work-up steps: filtration, concentration (e.g., concentration under reduced pressure, for another example, concentration under reduced pressure after adding ethyl acetate), cooling crystallization (e.g., cooling to -5-5 °C), washing (e.g., rinsing using ethyl acetate), and drying.

In some embodiments, when the hydrochloride form of compound A2 is obtained, the work-up step further comprises adding an acid (for example, adding hydrogen chloride in ethyl acetate, wherein the molar ratio of the hydrogen chloride in ethyl acetate to compound A1 is preferably 3:1-5:1, more preferably 4:1).

In some embodiments, the preparation method for compound A9 further comprises the following step: performing a reductive amination reaction of "compound B2 or the salt thereof" with tetrahydropyranone and acetic acid in a solvent in the presence of a reducing agent to obtain compound B3 or the salt thereof; 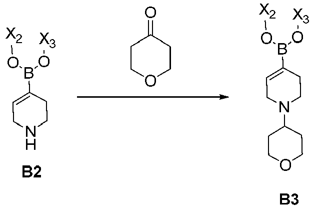 X₂ and X₃ are as defined in any aspect of the present disclosure.

In some embodiments, X₂ and X₃ are each independently H or C₁₋₆ alkyl.

In some embodiments, X₂ and X₃ are linked to form -C(CH₃)₂-C(CH₃)₂-, that is, 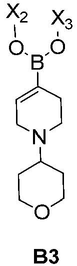 is 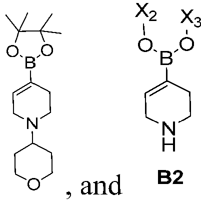 is

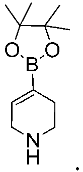

In some embodiments, the "salt of compound B2" is subjected to a reductive amination reaction with tetrahydropyranone and acetic acid.

In some embodiments, in the reductive amination reaction, the solvent is a halogenated alkane solvent, e.g., dichloromethane.

In some embodiments, the salt of compound B2 is the hydrochloride form of compound B2.

In some embodiments, when the salt of compound B2 is the hydrochloride form of compound B2, the molar ratio of compound B2 to hydrochloric acid is 1:1.

In some embodiments, the salt of compound B3 is the hydrochloride form of compound B3.

In some embodiments, when the salt of compound B3 is the hydrochloride form of compound B3, the molar ratio of compound B3 to hydrochloric acid is 1:1.

In some embodiments, in the reductive amination reaction, the reducing agent is sodium triacetoxyborohydride.

In some embodiments, in the reductive amination reaction, the molar ratio of the tetrahydropyranone to compound B2 is (2-5):1, e.g., 3.4:1.

In some embodiments, in the reductive amination reaction, the molar ratio of the acetic acid to compound B2 is (0.5-2):1, e.g., 1:1.

In some embodiments, in the reductive amination reaction, the molar ratio of the reducing agent to compound B2 is (2-5):1, e.g., 2.5:1.

In some embodiments, in the reductive amination reaction, the molar-to-volume ratio of compound B2 to the solvent is 1:(5-20) mol/L, e.g., 1:10 mol/L.

In some embodiments, the reductive amination reaction further comprises the following work-up steps: concentration (e.g., concentration under reduced pressure, or for another example, concentration under reduced pressure followed by adding methyl tert-butyl ether and concentrating under reduced pressure) and crystallization (e.g., crystallization in methyl tert-butyl ether);
further, the reductive amination reaction further comprises the following work-up steps: quenching (e.g., quenching using a 10% aqueous NaOH solution), filtration (e.g., filtration through celite), extraction (e.g., extraction using dichloromethane and water), drying (e.g., drying over anhydrous sodium sulfate), filtration, washing (e.g., washing using methyl tert-butyl ether), and drying.

In some embodiments, the reductive amination reaction is performed at room temperature (e.g., 15-25 °C).

In some embodiments, the reductive amination reaction is performed for 6-12 hours, e.g., 6 hours.

In some embodiments, the preparation method for compound A9 further comprises the following step: performing a deprotection reaction of compound B1 in a solvent under acidic conditions to obtain compound B2 or the salt thereof; 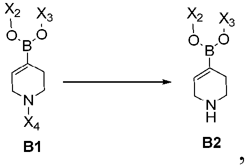 wherein X₄ is an amino protecting group; X₂ and X₃ are as defined in any aspect of the present disclosure.

In some embodiments, the amino protecting group is a group conventionally used in the art for protecting amino groups, such as -Boc.

In some embodiments, X₂ and X₃ are linked to form -C(CH₃)₂-C(CH₃)₂-, that is, 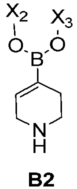 is

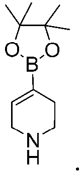

In some embodiments, X₂ and X₃ are each independently H or C₁₋₆ alkyl.

In some embodiments, 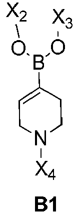 is

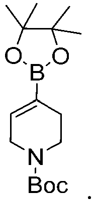

In some embodiments, compound B1 is subjected to a deprotection reaction to obtain the salt of compound B2.

In some embodiments, in the deprotection reaction, the solvent is an ester solvent, e.g., ethyl acetate.

In some embodiments, the salt of compound B2 is the hydrochloride form of compound B2.

In some embodiments, when the salt of compound B2 is the hydrochloride form of compound B2, the molar ratio of compound B2 to hydrochloric acid is 1:1.

In some embodiments, in the deprotection reaction, the acid in the acidic conditions is hydrogen chloride. The hydrogen chloride may be added in the form of a solution of hydrogen chloride in ethyl acetate. The concentration of the solution of hydrogen chloride in ethyl acetate may be 4 M. The mass ratio of the solution of hydrogen chloride in ethyl acetate to compound B1 is (3-10):1, e.g., 3.7:1.

In some embodiments, in the deprotection reaction, the mass ratio of compound B1 to the solvent is 1:(3-20), e.g., 1:3.2.

In some embodiments, the deprotection reaction is performed at room temperature (e.g., 25-35 °C).

In some embodiments, the deprotection reaction is performed for 2-6 hours.

In some embodiments, the deprotection reaction further comprises the following work-up steps: concentration under reduced pressure (e.g., concentrating the reaction liquid under reduced pressure to ≤ 2.5-3.5 v/w), crystallization (e.g., crystallization by stirring in methyl tert-butyl ether), filtration, washing (e.g., washing using methyl tert-butyl ether), and drying.

The present disclosure further provides a preparation method for compound A4, and the preparation method comprises the following reaction step: performing a coupling reaction between "A2 compound or a salt thereof" and "B3 compound or a salt thereof" in a solvent in the presence of a metal catalyst, a ligand, and a base to obtain compound A4, wherein the metal catalyst is a palladium catalyst; 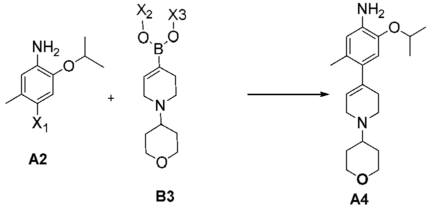
X₁ is halogen, e.g., F, Cl, Br, or I;
X₂ and X₃ are each independently H or C₁₋₆ alkyl; or, X₂ and X₃ are linked to form -C(CH₃)₂-C(CH₃)₂-.

In some embodiments, X₂ and X₃ are linked to form -C(CH₃)₂-C(CH₃)₂-, that is, 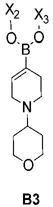 is

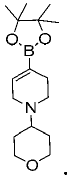

In some embodiments, the steps and the reaction conditions in the preparation method for compound A4 are as described in any aspect of the above.

The present disclosure further provides a preparation method for compound A2, and the preparation method comprises the following step: performing a reduction reaction of compound A1 in a solvent in the presence of a reducing agent to obtain compound A2 or a salt thereof; 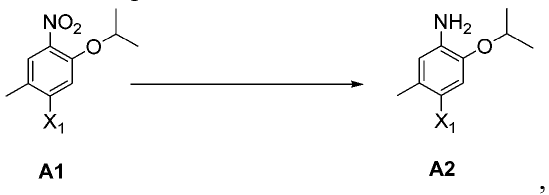
wherein X1 is F, Cl, Br, or I;
the reducing agent is iron powder and concentrated hydrochloric acid, hydrazine hydrate and ferric chloride, Pd/C and H₂, or Pt/C and H₂.

In some embodiments, the steps and the reaction conditions in the preparation method for compound A2 are as described in any aspect of the above.

The above preferred conditions may be combined arbitrarily to obtain preferred embodiments of the present disclosure without departing from the general knowledge in the art.

The reagents and starting materials used in the present disclosure are commercially available.

The positive and progressive effects of the present disclosure are as follows: The preparation method of the present disclosure has the advantages of appropriate reaction steps, simple and convenient operation, inexpensive and readily available reaction starting materials, environmental friendliness, simple and convenient work-up, low safety risk, and reduced production cycle and production cost.

### DETAILED DESCRIPTION

The present disclosure is further illustrated by the following examples, which are not intended to limit the present disclosure within the scope of the examples. Experimental methods without specified conditions in the following examples are conducted according to conventional methods and conditions, or according to product instructions.

In the following examples, w/w refers to the mass ratio of a substance to the reaction reference material; V refers to the volume-to-mass ratio of the solvent to the reaction reference material; and in the following examples, the reaction reference materials are B1', B2', B5a', B5', and B6', respectively.

### Example 1-Preparation Method for Intermediate B2'

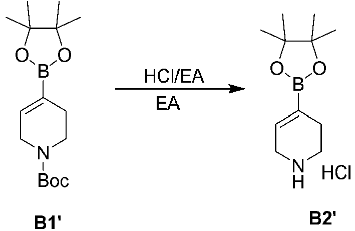

Ethyl acetate (357.55 kg, 3.2 w/w) was added to a reaction flask, stirring was started, and the reaction starting material B1' (111.40 kg, 1.0 w/w) was added. The mixture was warmed to 25-35 °C and stirred to dissolve the solid completely, and a 4 M HCl/EA solution (421.85 kg, 3.7 w/w) was added dropwise. After the dropwise addition, the temperature was controlled at 25-35 °C, and the mixture was stirred for 2-6 h. Samples were then taken for in-process control. After the reaction was completed, the reaction liquid was concentrated under reduced pressure to ≤ 2.5-3.5 v/w, and methyl tert-butyl ether (410.70 kg, 3.7 w/w) was added. The temperature of the system was controlled at 20-30 °C, and the mixture was stirred for crystallization for 6-10 h and filtered. The filter cake was washed with methyl tert-butyl ether (83.45 kg, 0.75 w/w) and dried to obtain 87.20 kg of product (yield: 99%, purity: 98.8%).

### Example 2-Preparation Method for Intermediate B4'

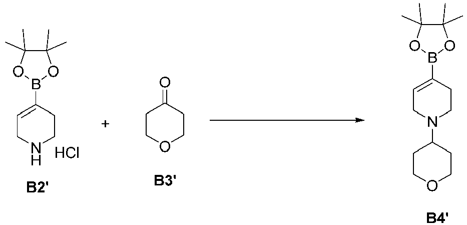

Dichloromethane (915.95 kg, 10.7 w/w), acetic acid (20.65 kg, 0.9 eq), B2' (85.26 kg, 1.0 eq), and B3' (119.60 kg, 3.4 eq) were sequentially added to a reaction flask, and the mixture was stirred uniformly. The temperature was controlled at 15 ± 5 °C, and sodium triacetoxyborohydride (185.70 kg, 2.5 eq) was added in batches. After the addition, the temperature was controlled at 20 ± 5 °C, and the mixture was stirred for 6 h. After the reaction was completed, the reaction liquid was added to a 10% aqueous NaOH solution to quench the reaction, and the resulting mixture was stirred, left to stand, and subjected to liquid separation. The organic phase was filtered (through celite), and the aqueous phase was extracted with dichloromethane. The organic phases were combined, anhydrous sodium sulfate (17.20 kg) was sequentially added, and the mixture was concentrated under reduced pressure. Methyl tert-butyl ether was then added, and the mixture was concentrated under reduced pressure. The temperature was controlled at 20 ± 5 °C, and methyl tert-butyl ether was added dropwise with stirring. After the dropwise addition, the mixture was cooled to -5-5 °C, stirred for crystallization for 2-4 h, and filtered. The filter cake was washed with methyl tert-butyl ether and dried to obtain 88.7 kg of product (yield: 87%, purity: 99.5%) as a white solid.

At the beginning of process development, the inventors found that, during the reduced-pressure concentration and purification steps of the work-up, when ethyl acetate and n-heptane were used for slurrying, the obtained product was granular and pale yellow, which was not conducive to product quality control. Therefore, a recrystallization method using methyl tert-butyl ether was developed to purify B4'.

### Example 3-Preparation of Intermediate B5'

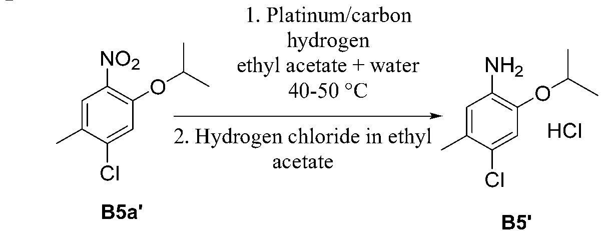

Ethyl acetate (587.1 kg, 9.0 w/w), B5a' (65.05 kg, 1.0 eq), 3% Pt/C (3.3 kg, 0.05 w/w), and purified water (1.90 kg, 0.029 w/w) were added to an autoclave, and the system was purged with nitrogen and then purged with hydrogen, with the hydrogen pressure controlled at 0.2-0.4 MPa and the temperature controlled at 40-50 °C. After 18-20 h of reaction, samples were taken for in-process control. After the reaction was completed, the system was cooled to 20-30 °C, and filtered to remove the platinum on carbon. The resulting mixture was concentrated under reduced pressure to a remaining volume of 3 V. The temperature was controlled at 15-30 °C, and hydrogen chloride in ethyl acetate (3.8 eq) was added dropwise to the system. After the dropwise addition, the mixture was stirred for 8-10 h with the temperature controlled at ≤ 50 °C, and then concentrated under reduced pressure to a remaining volume of 3 V. Ethyl acetate (3 V) was added to the system with the temperature controlled at ≤ 50 °C, and the mixture was concentrated under reduced pressure to a remaining volume of 3 V. The system was further cooled to -5-5 °C, stirred for 2-4 h, and filtered. The filter cake was rinsed with ethyl acetate and dried to obtain 60.20 kg of product (yield of the reaction: 90%, purity: 99.5%).

The inventors further investigated the hydrogen pressure, the stability of the reaction system at elevated temperature, the type of reducing agent, and the type of solvent, as follows:
(1) The inventors further screened the hydrogen pressure, and the results are as follows:

| Hydrogen pressure | Reaction time | Product purity |
|---|---|---|
| 1.0-1.5 MPa | 20 h | 89.84% |
| 0.4-0.6 MPa | 20 h | 99.42% |
| 0.2-0.4 MPa | 70 h | 99.43% |
| 0.05-0.30 MPa | 24 h | 99.19% |

As can be seen from the above table, when the hydrogen pressure was adjusted to 1.0-1.5 MPa and the reaction time was 20 h, the product purity was only 89.84%. Considering the relatively large scale of the production system, the reaction time might need to be extended. Under a pressure of 0.2-0.4 MPa, the reaction remained stable for 70 h, and no significant change in system purity was observed, and the purity still reached 99% or more.
(2) When the reaction system was allowed to react at 60-70 °C for 72 h, no significant change in reaction purity was observed, indicating that the reaction system was stable within 72 h under these conditions. (3) The inventors further optimized and adjusted the type of the reducing agent, and the results obtained are as follows.

| Type of reducing agent | Organic solvent in reaction system | Reaction time | Reaction temperature | Experimental result |
|---|---|---|---|---|
| Ferric chloride hexahydrate (0.02 w/w) + 80% hydrazine hydrate (0.625 w/w) | THF | 3 h | 60-65 °C | Yield: 78%, purity: 99.78% |
| Pd/C (0.05 w/w) + H₂ | Ethyl acetate | 24 h | 40-50 °C | Yield: 62%, purity: 95.28% |
| Pt/C (0.05 w/w) + H₂ | Ethyl acetate | 18-20 h | 40-50 °C | Yield: 90%, purity: 99.5% |
| Stannous chloride dihydrate + | Methanol | 4 h | 50 °C | Emulsification easily occurred during liquid |
| concentrated hydrochloric acid | | | | separation in the work-up, making phase separation difficult; thus, not suitable for industrial production. |

(4) The inventors further screened the type of solvent, and the results are as follows:

| Reaction solvent | Reaction temperature | Reaction time | Product purity |
|---|---|---|---|
| Methanol | 40-50 °C | 19 h | 98.14% |
| Isopropanol | 40-50 °C | 25 h | 97.64% |
| Ethyl acetate | 40-50 °C | 24 h | 98.98% |

### Example 4-Preparation Method for Intermediate B6'

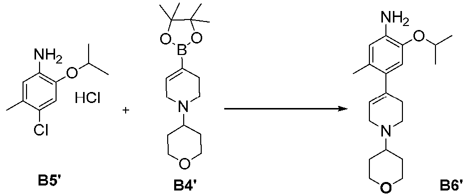

Isopropanol (653.0 kg, 11.9 w/w), water (55.10 kg, 1.0 w/w), and potassium phosphate (149.10 kg, 3.0 eq) were added to a reaction flask, and stirring was started. The temperature was controlled at 15-30 °C, and B4' (73.30 kg, 1.10 eq) and B5' (55.10 kg, 1.00 eq) were sequentially added. The system was purged with nitrogen with the temperature controlled at 15-30 °C, and palladium(II) acetate (0.525 kg, 0.01 eq) and RuPhos (2.10 kg, 0.02 eq) were added. The system was purged with nitrogen, warmed to 80 ± 5 °C, and allowed to react for 4-8 h. Samples were taken for in-process control. After the reaction was completed, the mixture was cooled to ≤ 50 °C, and purified water was added. The mixture was stirred for 0.5-1 h with the temperature controlled at 20-30 °C, followed by liquid separation. The organic phase was retained and concentrated. Ethyl acetate and water were then added, and the resulting mixture was left to stand and subjected to liquid separation. N-acetyl-L-cysteine (0.1 eq) and activated carbon (10% w/w) were added simultaneously, and the mixture was stirred at 50 °C for 1-3 h. The mixture was filtered and sequentially washed with an aqueous sodium carbonate solution and saturated brine, followed by concentration. At 20-30 °C, methyl tert-butyl ether and n-heptane were added dropwise with stirring, and the mixture was stirred at constant temperature for 1-2 h, followed by further concentration. n-Heptane was added, and the mixture was concentrated again, then cooled to -5-5 °C, and stirred for crystallization for 4 h. The mixture was filtered, and the filter cake was rinsed with n-heptane and dried to obtain 67.7 kg of product (yield of the reaction: 87%, purity: 99.4%).

With reference to the above preparation method, the type of ligand was optimized and adjusted, and the results obtained are as follows.

| Metal catalyst | Ligand | Base | Reaction solvent | Yield of reaction |
|---|---|---|---|---|
| Pd(OAc)₂ | Sphos | K₃PO₄ | toluene/H₂O | 83% |
| Pd(OAc)₂ | RuPhos | K₃PO₄ | toluene/H₂O | 87% |
| Pd(OAc)₂ | Xphos | K₂CO₃ | MeCN/H₂O | 83% |
| Pd(OAc)₂ | RuPhos | K₃PO₄ | MeCN/H₂O | 91% |
| Pd(OAc)₂ | PCy₃HBF₄ | K₃PO₄ | DMF/H₂O | 89% |

Further, the inventors optimized the palladium removal process in the work-up steps, and the results are as follows.

| Palladium removal process | Palladium residue in product |
|---|---|
| Activated carbon (0.1 w/w) for palladium removal | 1329 ppm |
| N-acetyl-L-cysteine (0.1 eq) for palladium removal, repeated 3 times | 316.4 ppm |
| Activated carbon (10%) and N-acetyl-L-cysteine (0.1 eq) were added simultaneously and stirred once (i.e., the procedure in the above preparation method) | 103.0 ppm |

When palladium is removed using N-acetyl-L-cysteine, the level of impurities in the system may increase. Therefore, the number of palladium removal treatments with N-acetyl-L-cysteine needs to be reduced. By combining the activated carbon treatment for palladium removal with the N-acetyl-L-cysteine treatment for palladium removal, the operation time can be shortened and the likelihood of emulsification during liquid separation can be reduced, while the Pd residue can be controlled to be below 200 ppm.

### Example 5-Preparation Method for Intermediate B8'

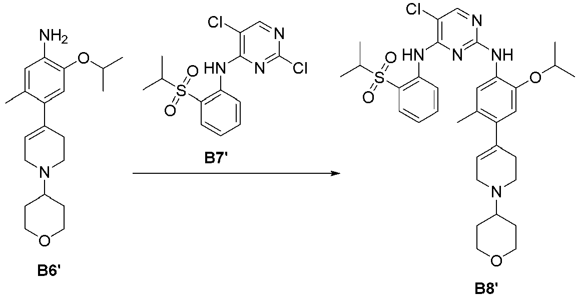

Under nitrogen atmosphere, B6' (30.1 kg, 1.0 eq), B7' (37.85 kg, 1.05 eq), and isopropanol (192.64 kg, 6.4 w/w) were added to a three-necked flask, and under nitrogen atmosphere, concentrated hydrochloric acid (1.2 eq) was added dropwise to the system. The mixture was stirred and warmed to 75-85 °C, and stirred for 24 h. After the reaction was completed, the mixture was cooled to 60-70 °C, and ethyl acetate (240 L, 8 V) was added dropwise. The system was cooled to room temperature, stirred at room temperature for 12-16 h, and filtered. The filter cake was washed with ethyl acetate and then collected.

Dichloromethane (180 L, 6 V) was added to the filter cake, and the mixture was stirred for 30 min. A 10% sodium carbonate solution was added under stirring, and the mixture was stirred for 30-60 min to ensure that the system was completely dissolved. The mixture was left to stand for phase separation, and the organic phase was collected. The organic phase was then washed with a 10% sodium carbonate solution, and the mixture was stirred for 30 min to ensure that the pH of the aqueous phase was > 8, and left to stand for phase separation. The organic phase was collected and washed, and the mixture was left to stand for phase separation. The organic phase was collected and concentrated under reduced pressure, and acetonitrile was added to the residue after concentration. The mixture was stirred overnight at room temperature and filtered. The filter cake was washed with acetonitrile and then collected.

Acetonitrile was added to the filter cake, and the resulting mixture was purged with nitrogen under stirring and maintained under nitrogen protection (note: protected from light).

The mixture was stirred and warmed to 75-85 °C, stirred for 0.5-1.5 h, then cooled to room temperature and stirred overnight, and filtered. The filter cake was washed with acetonitrile, collected, and dried under vacuum to obtain 51.78 kg of product (yield: about 89%, purity: 99.84%).

The procedures of Example 5 described above were repeated, except that the concentrated hydrochloric acid was replaced with p-toluenesulfonic acid, and the product yield was 74%. Since p-toluenesulfonic acid and alcohol solvents tend to form genotoxic impurities during heating, which is not conducive to subsequent process development, a process using concentrated hydrochloric acid as the acid catalyst is developed.

## Claims

1. A preparation method for compound A9, the preparation method comprising the following reaction steps: (1) performing a coupling reaction between "compound A2 or a salt thereof" and "compound B3 or a salt thereof" in a solvent in the presence of a metal catalyst, a ligand, and a base to obtain compound A4, wherein the metal catalyst is a palladium catalyst; 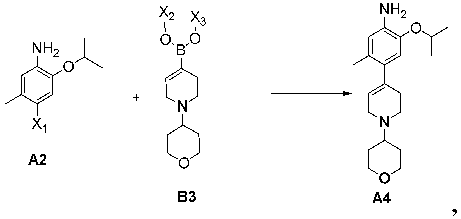 (2) performing a coupling reaction between compound A4 and compound A8 in a solvent in the presence of an acid to obtain compound A9; 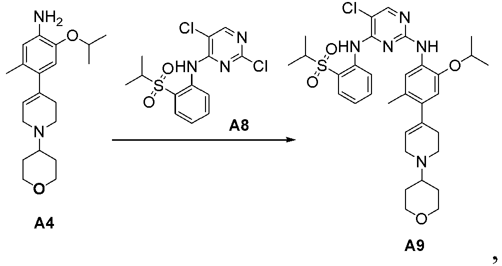
wherein X₁ is Cl, Br, or I;
X₂ and X₃ are each independently H or C₁₋₆ alkyl; or, X₂ and X₃ are linked to form -C(CH₃)₂-C(CH₃)₂-.

2. The preparation method according to claim 1, satisfying one or more of the following conditions:
(1) X₂ and X₃ are linked to form -C(CH₃)₂-C(CH₃)₂-, that is, 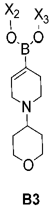 is 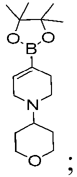
(2) X₁ is Cl or I, preferably Cl;
(3) the palladium catalyst is palladium(II) acetate, tetrakis(triphenylphosphine)palladium, or bis(triphenylphosphine)palladium (II) chloride, preferably palladium(II) acetate;
(4) in step (1), the solvent is one or more of an alcohol solvent, an ether solvent, an aromatic solvent, a nitrile solvent, a sulfoxide solvent, an amide solvent, and water, e.g., a combination of an alcohol solvent and water, a combination of an ether solvent and water, a combination of an aromatic solvent and water, a combination of a nitrile solvent and water, a combination of an amide solvent and water, an aromatic solvent, a nitrile solvent, a sulfoxide solvent, or an amide solvent, preferably a combination of an alcohol solvent and water, a combination of an aromatic solvent and water, a combination of a nitrile solvent and water, or a combination of an amide solvent and water;
(5) in step (1), the salt of compound A2 is a hydrochloride form of compound A2;
(6) in step (1), compound B3 is in a free form;
(7) in step (1), the base is an alkali metal carbonate and/or an alkali metal phosphate, for example, potassium carbonate and/or potassium phosphate, preferably potassium phosphate;
(8) in step (1), the ligand is a bidentate phosphine ligand or a biaryl phosphine ligand, e.g., BrettPhos, RuPhos, Xphos, SPhos, Bippyphos, CyPF-t-Bu, Josiphos, BINAP, tBuXphos, Me₄tBuXphos, tBuBrettPhos, DavePhos, JohnPhos, JackiePhos, (R)-(S)-Cy₂PF-PtBu₂, XantPhos, or PCy₃HBF₄, preferably RuPhos, Sphos, Xphos, or PCy₃HBF₄;
(9) in step (1), a molar ratio of compound B3 to compound A2 is (1-1.1):1, e.g., 1.1:1, 1.05:1, or 1.0:1;
(10) in step (1), a molar ratio of the metal catalyst to compound A2 is (0.01-0.02):1, e.g., 0.01:1 or 0.02:1;
(11) in step (1), a molar ratio of the base to compound A2 is (3.0-5.0):1, e.g., 3.0:1, 4.0:1, or 5.0:1;
(12) in step (1), a molar ratio of the ligand to compound A2 is 0.01:1-0.03:1;
(13) in step (1), the coupling reaction is performed at a reaction temperature of 80-120 °C;
(14) in step (1), the coupling reaction is performed for 4-8 hours;
(15) in step (1), the coupling reaction is performed under an inert gas atmosphere;
(16) in step (1), the coupling reaction further comprises a palladium removal step, for example, using one or more of N-acetyl-L-cysteine, activated carbon, and L-cysteine, preferably using N-acetyl-L-cysteine and activated carbon simultaneously for palladium removal;
(17) in step (1), the coupling reaction comprises the following work-up steps: liquid separation, concentration, extraction, palladium removal, filtration, washing, concentration, and recrystallization; and
(18) in step (1), the "salt of compound A2" and "compound B3" are subjected to a coupling reaction to obtain compound A4.

3. The preparation method according to claim 2, satisfying one or more of the following conditions:
(1) in step (1), the alcohol solvent is isopropanol;
(2) in step (1), the ether solvent is dioxane and/or methyl ether;
(3) in step (1), the aromatic solvent is toluene;
(4) in step (1), the nitrile solvent is acetonitrile;
(5) in step (1), the sulfoxide solvent is dimethyl sulfoxide;
(6) in step (1), the amide solvent is N,N-dimethylformamide;
(7) in step (1), when the solvent is a combination of an alcohol solvent and water, a mass ratio of the alcohol solvent to the water is (4-20):1, preferably 11.9:1;
(8) in step (1), when the salt of compound A2 is the hydrochloride form of compound A2, a molar ratio of compound A2 to hydrochloric acid is 1:1;
(9) in step (1), the ligand is RuPhos;
(10) in step (1), a molar ratio of compound B3 to compound A2 is 1.1:1;
(11) in step (1), a molar ratio of the metal catalyst to compound A2 is 0.01:1;
(12) in step (1), a molar ratio of the base to compound A2 is 3.0:1;
(13) in step (1), a molar ratio of the ligand to compound A2 is 0.02:1;
(14) in step (1), when the solvent is a combination of an alcohol solvent and water, a volume-to-molar ratio of the water to compound A2 is 0.20 L/mol-0.40 L/mol, preferably 0.35 L/mol;
(15) in step (1), the coupling reaction is performed at a reaction temperature of 75-85 °C;
(16) in step (1), the coupling reaction is performed for 6 hours;
(17) in step (1), the coupling reaction is performed under nitrogen atmosphere; and
(18) in step (1), when the coupling reaction further comprises a step of simultaneously using N-acetyl-L-cysteine and activated carbon for palladium removal, a molar ratio of N-acetyl-L-cysteine to compound A2 is 0.1:1; a mass percentage of the activated carbon in the reaction system is 10%.

4. The preparation method according to claim 1, satisfying one or more of the following conditions:
(1) in step (2), the solvent is an alcohol solvent;
(2) in step (2), the acid is an organic strong acid or an inorganic strong acid; the organic strong acid may be 2,4,6-trinitrophenol, 2,4,6-trinitrobenzoic acid, trifluoroacetic acid, trichloroacetic acid, methanesulfonic acid, benzenesulfonic acid, cyclohexylthiolsulfonic acid, or p-toluenesulfonic acid, preferably p-toluenesulfonic acid; the inorganic strong acid may be concentrated hydrochloric acid, an organic solution of hydrogen chloride, perchloric acid, hydroiodic acid, hydrobromic acid, sulfuric acid, or nitric acid, preferably concentrated hydrochloric acid;
(3) in step (2), a molar ratio of compound A8 to compound A4 is (1-1.5):1;
(4) in step (2), a molar ratio of the acid to compound A4 is (1-1.5):1;
(5) in step (2), the molar-to-volume ratio of compound A4 to the solvent is 1:(6-15) mol/L;
(6) in step (2), the coupling reaction is performed at a temperature of 70-90 °C;
(7) in step (2), the coupling reaction is performed under an inert gas atmosphere;
(8) in step (2), the coupling reaction is performed for 12-24 hours; and
(9) in step (2), the coupling reaction further comprises the following work-up steps: crystallization, filtration, washing, liquid separation, washing of organic phase, concentration under reduced pressure, crystallization, and drying.

5. The preparation method according to claim 4, satisfying one or more of the following conditions:
(1) when the inorganic strong acid is an organic solution of hydrogen chloride, the organic solution of hydrogen chloride is a solution of hydrogen chloride in isopropanol, hydrogen chloride in ethyl acetate, hydrogen chloride in methanol, hydrogen chloride in ethanol, or hydrogen chloride in dioxane;
(2) in step (2), the molar ratio of compound A8 to compound A4 is 1.05:1;
(3) in step (2), the molar ratio of the acid to compound A4 is 1.2:1;
(4) in step (2), the molar-to-volume ratio of compound A4 to the solvent is 1:8 mol/L;
(5) in step (2), the coupling reaction is performed at a temperature of 75-85°C;
(6) in step (2), the coupling reaction is performed under nitrogen atmosphere; and
(7) in step (2), the coupling reaction is performed for 24 hours.

6. The preparation method according to claim 1, wherein the preparation method for compound A9 further comprises the following step: performing a reduction reaction of compound A1 in a solvent in the presence of a reducing agent to obtain compound A2 or the salt thereof; 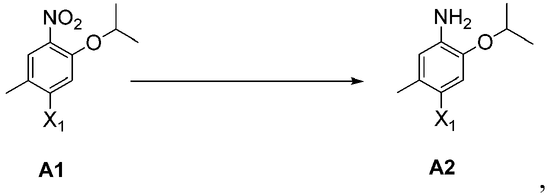 wherein X₁ is Cl, Br, or I.

7. The preparation method according to claim 6, satisfying one or more of the following conditions:
(1) the reducing agent is iron powder and concentrated hydrochloric acid, stannous chloride dihydrate and concentrated hydrochloric acid, hydrazine hydrate and ferric chloride, Pd/C and H₂, or Pt/C and H₂;
(2) in the reduction reaction, a hydrogen pressure of the reaction system is 0.05-0.6 MPa, preferably 0.2-0.4 MPa, more preferably normal pressure;
(3) in the reduction reaction, the reaction system is first purged with nitrogen and then purged with hydrogen;
(4) in the reduction reaction, the solvent is an organic solvent or a combination of an organic solvent and water, and the organic solvent is an ether solvent, an ester solvent, or an alcohol solvent; preferably, the solvent is an ester solvent or a combination of an ester solvent and water; more preferably, the solvent is ethyl acetate or a combination of ethyl acetate and water;
(5) in the reduction reaction, a molar-to-volume ratio of compound A1 to the organic solvent is (1-3):10 mol/L;
(6) in the reduction reaction, when the solvent is a combination of an organic solvent and water, a mass ratio of the water to compound A1 is 2%-4%;
(7) in the reduction reaction, when the reducing agent is Pt/C and hydrogen, the Pt/C is 3% Pt/C;
(8) in the reduction reaction, a mass percentage of the reducing agent to compound A1 is 4%-6%;
(9) the salt of compound A2 is a hydrochloride form of compound A2;
(10) the reduction reaction is performed at a temperature of 40-70 °C;
(11) the reduction reaction is performed for 18-20 h; and
(12) the reduction reaction further comprises the following work-up steps: filtration, concentration, cooling crystallization, washing, and drying.

8. The preparation method according to claim 7, satisfying one or more of the following conditions:
(1) in the reduction reaction, the ether solvent is tetrahydrofuran;
(2) in the reduction reaction, the ester solvent is ethyl acetate;
(3) in the reduction reaction, the alcohol solvent is methanol or isopropanol;
(4) in the reduction reaction, the molar-to-volume ratio of compound A1 to the organic solvent is 2:10 mol/L;
(5) in the reduction reaction, when the solvent is a combination of an organic solvent and water, the mass ratio of the water to compound A1 is 3%;
(6) in the reduction reaction, the mass percentage of the reducing agent to compound A1 is 5%;
(7) when the salt of compound A2 is the hydrochloride form of compound A2, a molar ratio of compound A2 to hydrochloric acid is 1:1;
(8) the reduction reaction is performed at a temperature of 40-50 °C;
(9) the reduction reaction is performed for 20 h; and
(10) when the hydrochloride form of compound A2 is obtained, the work-up step further comprises adding an acid, for example, adding hydrogen chloride in ethyl acetate, wherein a molar ratio of the hydrogen chloride in ethyl acetate to compound A1 is preferably 3:1-5:1, more preferably 4:1.

9. The preparation method according to claim 1, wherein the preparation method for compound A9 further comprises the following step: performing a reductive amination reaction of "compound B2 or the salt thereof" with tetrahydropyranone and acetic acid in a solvent in the presence of a reducing agent to obtain compound B3 or the salt thereof;

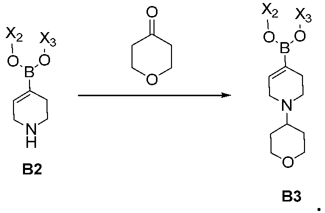

10. The preparation method according to claim 9, satisfying one or more of the following conditions:
(1) in the reductive amination reaction, the solvent is a halogenated alkane solvent;
(2) the salt of compound B2 is a hydrochloride form of compound B2;
(3) the compound B3 is in a free form;
(4) in the reductive amination reaction, the reducing agent is sodium triacetoxyborohydride;
(5) in the reductive amination reaction, a molar ratio of the tetrahydropyranone to compound B2 is (2-5):1;
(6) in the reductive amination reaction, a molar ratio of the acetic acid to compound B2 is (0.5-2):1;
(7) in the reductive amination reaction, a molar ratio of the reducing agent to compound B2 is (2-5):1;
(8) in the reductive amination reaction, a molar-to-volume ratio of compound B2 to the solvent is 1:(5-20) mol/L;
(9) the reductive amination reaction further comprises the following work-up steps: concentration and crystallization;
(10) the reductive amination reaction is performed at room temperature; and
(11) the reductive amination reaction is performed for 6-12 h.

11. The preparation method according to claim 10, satisfying one or more of the following conditions:
(1) in the reductive amination reaction, the solvent is dichloromethane;
(2) when the salt of compound B2 is the hydrochloride form of compound B2, a molar ratio of compound B2 to hydrochloric acid is 1:1;
(3) in the reductive amination reaction, the molar ratio of the tetrahydropyranone to compound B2 is 3.4:1;
(4) in the reductive amination reaction, the molar ratio of the acetic acid to compound B2 is 1:1;
(5) in the reductive amination reaction, the molar ratio of the reducing agent to compound B2 is 2.5:1;
(6) in the reductive amination reaction, the molar-to-volume ratio of compound B2 to the solvent is 1:10 mol/L;
(7) in the reductive amination reaction, in the work-up steps, the concentration is concentration under reduced pressure, for example, concentration under reduced pressure followed by adding methyl tert-butyl ether and concentrating under reduced pressure;
(8) in the reductive amination reaction, in the work-up steps, the crystallization is crystallization in methyl tert-butyl ether;
(9) the reductive amination reaction further comprises the following work-up steps: quenching, filtration, extraction, drying, filtration, washing, and drying;
(10) the reductive amination reaction is performed at a temperature of 15-25 °C;
(11) the reductive amination reaction is performed for 6 h; and
(12) the "salt of compound B2" is subjected to a reductive amination reaction with tetrahydropyranone and acetic acid.

12. The preparation method according to claim 9, wherein the preparation method for compound A9 further comprises the following step: performing a deprotection reaction of compound B1 in a solvent under acidic conditions to obtain compound B2 or the salt thereof; 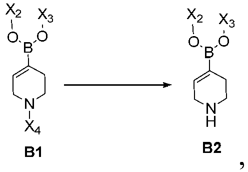 wherein X₄ is an amino protecting group.

13. The preparation method according to claim 12, satisfying one or more of the following conditions:
(1) the amino protecting group is -Boc;
(2) in the deprotection reaction, the solvent is an ester solvent;
(3) the salt of compound B2 is a hydrochloride form of compound B2;
(4) in the deprotection reaction, an acid in the acidic conditions is hydrogen chloride; the hydrogen chloride may be added in a form of a solution of hydrogen chloride in ethyl acetate;
(5) in the deprotection reaction, a mass ratio of compound B1 to the solvent is 1:(3-20);
(6) the deprotection reaction is performed at room temperature;
(7) the deprotection reaction is performed for 2-6 hours; and
(8) the deprotection reaction further comprises the following work-up steps: concentration under reduced pressure, crystallization, filtration, washing, and drying.

14. The preparation method according to claim 13, satisfying one or more of the following conditions:
(1) in the deprotection reaction, the solvent is ethyl acetate;
(2) when the salt of compound B2 is the hydrochloride form of compound B2, a molar ratio of compound B2 to hydrochloric acid is 1:1;
(3) a concentration of the solution of hydrogen chloride in ethyl acetate may be 4 M; a mass ratio of the solution of hydrogen chloride in ethyl acetate to compound B1 is (3-10):1, e.g., 3.7:1;
(4) in the deprotection reaction, the mass ratio of compound B1 to the solvent is 1:3.2; and
(5) the deprotection reaction is performed at a reaction temperature of 25-35 °C.

15. A preparation method for compound A4, the preparation method comprising the following reaction step: performing a coupling reaction between "A2 compound or a salt thereof" and "B3 compound or a salt thereof" in a solvent in the presence of a metal catalyst, a ligand, and a base to obtain compound A4, wherein the metal catalyst is a palladium catalyst; 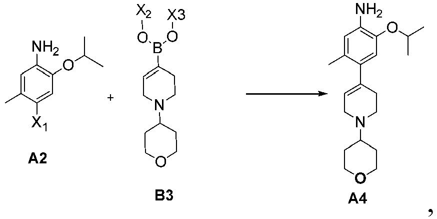
wherein X₁ is Cl, Br, or I;
X₂ and X₃ are each independently H or C₁₋₆ alkyl; or, X₂ and X₃ are linked to form -C(CH₃)₂-C(CH₃)₂-;
the steps and the reaction conditions in the preparation method for compound A4 are as described in any one of claims 1-3.

16. A preparation method for compound A2, the preparation method comprising the following step: performing a reduction reaction of compound A1 in a solvent in the presence of a reducing agent to obtain compound A2 or a salt thereof; 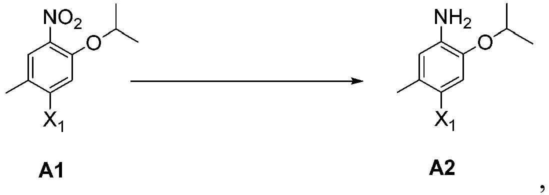
wherein X₁ is Cl, Br, or I;
the reducing agent is iron powder and concentrated hydrochloric acid, hydrazine hydrate and ferric chloride, Pd/C and H₂, or Pt/C and H₂;
the steps and the reaction conditions in the preparation method for compound A2 are as described in any one of claims 6-8.
